# EUROPEAN PATENT SPECIFICATION

(11) **EP 0 183 439 B1**
(45) Date of publication and mention of the grant of the patent: **03.02.1993**
(21) Application number: 85308279.0
(22) Date of filing: 14.11.1985
(51) Int. Cl.: C07D 333/76, C07D 333/74, C07D 333/78, C07D 307/91, C07D 307/92, C07D 219/02, C07D 215/14, A61K 31/40, A61K 31/38, A61K 31/435

(54) **Polycyclic compounds, their preparation and use and formulations containing them**
Polycyclische Verbindungen, ihre Herstellung und Verwendung und sie enthaltende Rezepturen
Composés polycycliques, leur préparation et leur utilisation et formulations les contenant

(30) Priority: 15.11.1984 GB 8428930
(43) Date of publication of application: 04.06.1986
(73) Proprietor: THE WELLCOME FOUNDATION LIMITED, London NW1 2BP (GB)
(72) Inventor: Bair, Kenneth Walter, Chapel Hill North Carolina 27514 (US)
(74) Representative: Rollins, Anthony John (GB)

(56) References cited:
- EP-A- 0 125 701
- EP-A- 0 125 702
- DE-A- 2 510 001
- US-A- 4 219 657
- ARZNEIMITTELFORSCHUNG/Drug Research, vol. 9, 1982, pages 1013-1016; M. HRABOESKA et al.: "Antitumor Activity of 1-Nitro-9-aminoacridine Derivatives"

## Description

The present invention relates to heteropolycyclic aromatic alkanol derivatives which have been found to have biocidal activity. More specifically the invention concerns aminoalkanol derivatives containing a heteropolycyclic aromatic ring system, methods for the synthesis thereof, pharmaceutical formulations thereof, novel intermediates therefor, pharmaceutical formulations thereof and the use thereof as biocidal agents, particularly antitumor agents.

Two analogues of nitracrine (1-nitro-9-((3-dimethylaminopropyl)amino)-acridine) containing 2-amino-2-methyl-1,3-propanediol and tris(hydroxymethyl)methylamine groups have been reported to have antitumor activity in some murine screening systems (Arzneim. Forsch./Drug Res. 32II, 1013 (1982)).

We have now discovered a novel class of heteropolycyclic aromatic alkanol derivatives which have biocidal activity.
Accordingly, in a first aspect, the present invention provides a compound of the formula (I):

R CH₂NHR¹ (I)

or a monomethyl or monoethyl ether thereof, the compound of formula (I) including its ethers containing no more than 29 carbon atoms in total, or an ester or a salt thereof;
wherein R is is a fused ring system containing 3,4 or 5 rings and a maximum of 4n+1 carbon atoms, n being the number of rings present, at least three rings being aromatic when n is 3 or 4 and at least four rings being aromatic when n is 5, at least one of the aromatic rings containing a heteroatom; optionally substituted by one or two substituents; said substituents containing not more than four carbon atoms in total when taken together and being the same or different and are selected from halogen; cyano; C₁₋₄ alkyl or C₁₋₄ alkoxy, each optionally substituted by hydroxy or C₁₋₂ alkoxy; halogen substituted C₁₋₂ alkyl or C₁₋₂ alkoxy; a group S(O)_{n'}R² wherein n' is an integer, 0,1 or 2 and R² is C₁₋₂ alkyl optionally substituted by hydroxy or C₁₋₂ alkoxy; or R is optionally substituted by a group NR³R⁴ containing not more than 5 carbon atoms wherein R³ and R⁴ are the same or different and each is a C₁₋₃ alkyl group or NR³R⁴ forms a five-or six-membered heterocyclic ring optionally containing one or two additional heteroatoms;
R¹ is 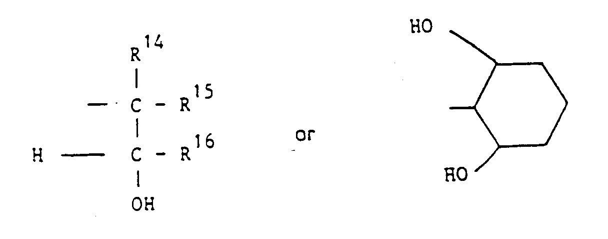
wherein R¹⁴ is CH₂OH,CH(CH₃)OH or CH₂CH₂OH,
R¹⁵ is hydrogen, C₁₋₃ alkyl or CH₂OH
R¹⁶ is hydrogen or methyl;
Preferably R¹⁴ is CH₂OH or CH(CH₃)OH; Preferably R¹⁵ is hydrogen, methyl, ethyl or CH₂OH,
except that R is not a tetracyclic ring system containing 15 to 17 ring atoms, three six membered carbocyclic rings and one five membered ring in which there is a heteroatom.

There is a maximum of one heteroatom for each aromatic ring present but preferably only one or two of the aromatic rings contains a heteroatom. The rings contain five or six atoms and the heteroatom is conveniently nitrogen, phosphorus, oxygen, sulphur or selenium and is suitably oxygen, nitrogen or sulphur. Preferably the ring system is planar or deviates only slightly from planarity. Suitably the ring system is aromatic or contains one non-aromatic ring. Preferably the ring sytem is aromatic. In the case of a five membered ring containing nitrogen the nitrogen is substituted by hydrogen, methyl or ethyl.

Preferred 3-ringed aromatic heterocycles include 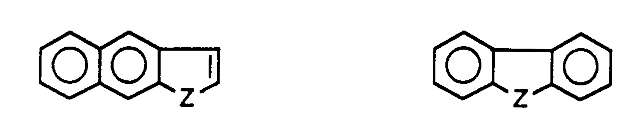
wherein Z = sulphur, oxygen, NMe or NEt. Preferred 4-ring aromatic heterocycles include 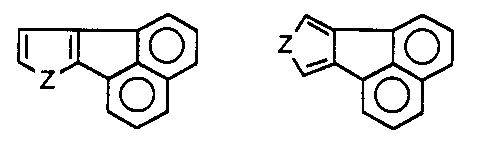
wherein Z = sulphur, oxygen, NMe or NEt
Suitably the aromatic ring system is optionally substituted by one substituent.

Suitably R CH₂NHR¹ or a monomethyl or monethyl ether thereof contains not more than 28 carbon atoms in total.

Preferably R¹ is 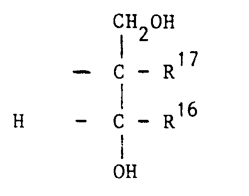
wherein R¹⁶ is hydrogen or methyl and R¹⁷ is hydrogen, methyl or ethyl, most preferably methyl.

Preferred compounds of the present invention include:
2-[(9-Acridinylmethyl)amino]-2-methyl-1,3-propanediol,
2-[(1-Dibenzothiophenylmethyl)amino]-2-methyl-1,3-propanediol,
2-[(2-Dibenzothiophenylmethyl)amino]-2-methyl-1,3-propanediol,
2-[(4-Dibenzothiophenylmethyl)amino]-2-methyl-1,3-propanediol,
2-[(1-Dibenzofuranylmethyl)amino]-2-methyl-1,3-propanediol,
2-[(2-Dibenzofuranylmethyl)amino]-2-methyl-1,3-propanediol
2-[(Naphtho[2,3-b]thiophen-4-ylmethyl)amino]-2-methyl-1,3-propanediol,
2-Methyl-2-[(naphtho[2,1-b]thiophen-5-ylmethyl)amino]-1,3-propanediol,
2-Methyl-2-[(naphtho[2,1-b]thiophen-2-ylmethyl)amino]-1,3-propanediol,
2-Methyl-2-[(naphtho[1,2-b]thiophen-2-ylmethyl]-1,3-propanediol,
2-Methyl-2-[(naphtho[1,2-b]thiophen-5-ylmethyl)amino]-1,3-propanediol,
2-Methyl-2-[(naphtho[1,2-b]furan-2-ylmethyl)amino]-1,3-propanediol,
2-Methyl-2-[(naphtho[2,1-b]furan-2-ylmethyl)amino]-1,3-propanediol,
2-[(Acenaphtho[1,2-b]quinolin-10-ylmethyl)amino]-2-methyl-1,3-propanediol,
2-[(Acenaphtho[1,2-b]thiophen-8-ylmethyl)amino]-2-methyl-1,3-propanediol,
2-[Acenaphtho[1,2-c]thiophen-7-ylmethyl)amino]-2-methyl-1,3-propanediol.

Salts included within the scope of the present invention are those of the compounds of formula (I) and ethers and esters thereof.

Esters and non-pharmaceutically useful salts of the compounds of the formula (I) are useful intermediates in the preparation and purification of compounds of the formula (I) and pharmaceutically useful salts thereof, and are therefore within the scope of the present invention. Thus, salts of the compounds of the formula (I) useful in the present invention include but are not limited to those derived from inorganic acids, such as hydrochloric, hydrobromic, sulfuric and phosphoric acids, and organic acids such as isethionic (2-hydroxyethylsulfonic), maleic, malonic, succinic, salicylic, tartaric, lactic, citric, formic, lactobionic, pantothenic, methanesulfonic, ethanesulfonic, benzenesulfonic, p-toluenesulfonic, naphthalene-2-sulfonic, and ascorbic acids, and amino acids such as glycine.

Pharmacologically and pharmaceutically acceptable salts are preferred, particularly those that are soluble in solvents suitable for parenteral administration, for example, hydrochlorides, methanesulfonates and isethionates.

Esters of compounds of formula (I) are derived from acids known to those skilled in the art to be suitable for ester formation, and are conveniently those derived from C₁₋₆ alkanoic acids or alkanoic acid derivatives, for example acetic acid, propionic acid, n-butyric acid and iso-butyric acid. The esters may be formed from all or only some of the hydroxy groups contained in the compounds of formula (I). Specific compounds within the scope of formula (I) include;
and ethers, esters, and salts thereof.

The compounds of formula (I) and their ethers, esters, and salts thereof may be prepared by any method known in the art for the preparation of compounds of analogous structure. Thus, the compounds of formula (I) may, for example, be prepared by any of the methods defined below.
1. The reduction of a compound R-CH=N-R¹
   wherein R and R¹ are as hereinbefore defined or an appropriately protected derivative thereof followed by deprotection where appropriate.
   The conditions and reagents for such a reaction are well known to those skilled in the art, and any such conditions/reagents may be employed that will not reduce the aromatic ring system. The conversion of (II) or suitably protected derivatives thereof may be carried out by a reducing agent followed by deprotection if necessary. The reduction is conveniently carried out by a metal hydride such as lithium aluminum hydride, sodium borohydride, sodium cyanoborohydride, or by catalytic hydrogenation, conveniently by hydrogen in the presence of a metal catalyst such as palladium or platinum, or equivalent reagents as outlined by J. March, Advanced Organic Chemistry, 2nd ed., pages 819-820, McGraw Hill, New York, 1977. The reduction is suitably carried out with the compound of formula (II) in solution in an inert solvent or mixture of solvents compatible with the reducing agent, at a non-extreme temperature, for example, between 0° and 80°C, conveniently at room temperature.
   In the case of lithium aluminum hydride and like reagents, suitable solvents include ethers (for example tetrahydrofuran, diethyl ether and 1,2-dimethoxy ethane) optionally in the presence of a hydrocarbon cosolvent (for example toluene, benzene or hexane).
   In the case of sodium borohydride and like reagents, suitable solvents include alcohols (for example ethanol, methanol or isopropanol) optionally in the presence of a hydrocarbon cosolvent (for example toluene, benzene or hexane) or an ether cosolvent (for example diethylether or tetrahydrofuran).
   In the case of sodium cyanoborohydride and like reagents, suitable solvents include those described for sodium borohydride and the reduction is conveniently carried out in the presence of an acid, conveniently glacial acetic acid or ethanolic hydrochloric acid as outlined in, for example, R. Hutchins et al., Organic Preparations and Procedures International 11, 201 (1979).
   In the case of catalytic hydrogenation, suitable solvents include alcohols (for example methanol and ethanol) optionally in the presence of a hydrocarbon cosolvent (for example toluene or benzene), or ether cosolvent (for example diethyl ether or tetrahydrofuran) optionally in the presence of an acid (for example glacial acetic acid or ethanolic hydrochloric acid), or glacial acetic acid.
   Protected derivatives of compounds R CH=NR¹ are conveniently used when lithium aluminum hydride is employed as the reducing agent. Convenient protecting groups are compatible with the reducing agent utilized and are readily removed under nondestructive conditions, for example benzyl, tetrahydropyranyl and isopropylidene ethers.
   It is often convenient not to isolate the compound R CH=NR¹ but to react a compound RCHO with a compound NH₂R¹ wherein R and R¹ are as defined in (I) and to reduce the compound RCH=NR¹ so formed in situ. The reaction of the compounds RCHO and NH₂R¹ is again suitably carried out using conditions and reagents which are well known to those skilled in the art, for example in the presence of an acid, such as a sulfonic acid, i.e. p-toluenesulfonic acid, in an appropriate inert solvent, such as an aromatic hydrocarbon, suitably toluene, with azeotropic removal of water followed by treatment with the reducing agent in an appropriate solvent, suitably ethanol or methanol. Alternatively, RCH=NR¹ formed under equilibrium conditions in appropriate solvents can be reduced in situ with an appropriate reducing agent, suitably sodium cyanoborohydride.
   The compound RCHO may be in the form of a protected aldehyde, for example an acetal, which liberates the aldehyde function under the reaction conditions. In turn, RCHO can be synthesized by reacting the appropriate aromatic polyheterocycle with a formylating agent such as that generated by the reaction between SnCl₄ and Cl₂CHOCH₃ or equivalent reagents, for example, according to the method of A. Reiche et al., Chem. Ber. 93, 88 (1960), or with other standard formylating reagents/procedures known to the art, for example, the Gatterman-Koch reaction (CO/HCl/AlCl₃/CuCl), the Gatterman reaction (HCN/HCl/ZnCl₂), and the Vilsmeier reaction (POCl₃/PhN(Me)CHO or POCl₃/Me₂NCHO) (J. March, vide supra pages 494-497).
   The compounds RCHO may also be prepared from an appropriate aromatic heterpolycycle substituted by a suitable functional group and converting this functional group to an aldehyde group by methods well known to those skilled in the art. Suitable functional groups include CHBr₂, CH₃, COR¹⁹ wherein R¹⁹ is a primary or secondary C₁₋₆ alkyl group, COOH or a derivative thereof such as an ester, amide or acid chloride or CN.
   Where the aromatic polyheterocycle bears substituents, RCHO may be prepared by a variety of methods known in the art of organic chemistry depending on the nature of the substituent on the polycyclic ring. For example, if the substituent(s) is a halogen, the starting materials may be prepared by direct treatment of the polycyclic aromatic hydrocarbon with a halogenating agent (e.g. Cl₂, Br₂, or SO₂Cl₂) or indirectly by such routes as the Sandmeyer reaction (H.H. Hodgson, Chem. Rev. 40, 251 (1947). If the substituent(s) is alkyl, the aromatic polyheterocycle may be reacted with the appropriate reagents under Friedel-Crafts reaction conditions (G.A. Olah, Friedel Crafts and Related Reactions, Vols. 1-3, Interscience, New York, NY, 1963-1965).
   The compounds NH₂R¹ also may be prepared by methods known in the art, for example when R¹ is as hereinbefore defined, by the reaction of compound NO₂R¹⁹ wherein R¹⁹ is a group 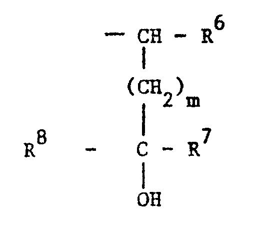 with an appropriate aldehyde, conveniently acetaldehyde or formaldehyde (as in B.M. Vanderbilt and H.B. Hass, Ind. Eng. Chem. 32, 34 (1940)) followed by reduction (as outlined in J. March, vide supra, pages 1125-1126), conveniently by hydrogen and a metal catalyst (for example, a platinum containing catalyst) in an appropriate solvent, conveniently glacial acetic acid.
2. The reduction of a compound R. CO. NHR¹
   wherein R and R¹ are as hereinbefore defined and the hydroxy groups are optionally protected, followed by deprotection of the hydroxy groups where appropriate. The reduction may be carried out by standard reducing agents known for carrying out this type of reduction that will not reduce the aromatic ring system (as outlined in J. March, vide supra, page 1122), for example, a hydride reagent such as lithium aluminium hydride in an inert solvent, such as an ether, i.e. tetrahydrofuran, at a non-extreme temperature, for example, at between 0^{o} and 100^{o}C and conveniently at the reflux temperature of the ether. The compound R. CO. NHR¹ may be formed by the reaction of the appropriate acid (RCOOH) or a suitable reactive acid derivative thereof as outlined in J. March, vide supra, pages 382-390) for example, an acid halide in an inert solvent, with an amine NH₂R¹ in which the hydroxy groups are optionally protected, for example, when the compound NH₂R¹ is a diol, by an isopropylidene group. The compound R. CO. NHR¹ so formed is suitably reduced in situ and deprotected if necessary to give a compound of formula (I). The compounds of the formula RCOOH can be prepared by methods well known to those skilled in the art.
3. The reaction of a compound RCH₂L (wherein R is as hereinbefore defined and L is a leaving group), with a compound NH₂R¹ as hereinbefore defined. Suitable leaving groups are those defined by J. March, vide supra pages 325-331, and include halogens such as chlorine or bromine and sulfonic acid derivatives such as p-toluenesulfonate. The reaction is suitably carried out in an appropriate solvent, such as a dipolar aprotic solvent or alcohol at a non-extreme temperature, for example 50-100^{o}. The compounds of the formula RCH₂L can be prepared by methods well known to those skilled in the art.

There is therefore provided, as a further aspect of the invention, a method for the preparation of a compound of formula (I) comprising any method known for the preparation of analogous compounds, in particular those methods defined in (1) to (3) hereinabove.

Compounds of the formula (I) in which one or more hydroxy group are protected, for example by benzyl or trityl groups or by an isopropylidene group are also useful intermediates in the preparation of compounds of the present invention.

The compounds of this invention have biocidal activity, e.g. are toxic to certain living cells which are detrimental to mammals, for example pathogenic organisms and tumour cells.

This toxicity to pathogenic organisms has been demonstrated by activity against viruses (e.g. Herpes simplex l/vero), fungi (e.g. Candida albicans), protozoa (e.g. Eimeria tenella and Trichomonas vaginalis), bacteria (e.g. Mycoplasma smegmatis and Streptococcus pyogenes), and helminths (e.g. Nippostrongylus brasiliensis and Brugia pahangi). The antitumour activity of compounds of formula I has been demonstrated in a number of recognized screens and primarily by activity against ascitic P388/O leukaemia.

Preferred compounds of the formula (I) are those which have antitumour activity. The activity against ascitic tumours, including P388/O, is evidenced by reduction of tumour cell number in mammals (for example, mice bearing ascitic tumours) and their consequent increase in survival duration as compared to an untreated tumour bearing control group. Antitumour activity is further evidenced by measurable reduction in the size of solid tumours following treatment of mammals with the compounds of this invention compared to the tumours of untreated control tumour bearing animals. Compounds of formula (I) are active against murine tumours such as lymphocytic leukaemia P388/0, lymphocytic leukaemia L1210, melanotic melanoma B16, P815 mastocytoma, MDAY/D2 fibrosarcoma, colon 38 adenocarcinoma, M5076 rhabdomyosarcoma and Lewis lung carcinoma.

Activity in one or more of these tumour tests has been reported to be indicative of antitumour activity in man (A. Goldin et al. in Methods in Cancer Research ed. V.T. DeVita Jr. and H. Busch, 16, 165, Academic Press, N.Y. 1979).

There are sublines of P388/0 which have been made resistant to the following clinically useful agents: cytosine arabinoside, doxorubicin, cyclophosphamide, L-phenylalanine mustard, methotrexate, 5-fluorouracil, actinomycin D, cis-platin and bis-chloroethylnitrosourea. Compounds of this invention show potent acti vity against these drug-resistant tumours using the procedure for P388/O above.

Compounds of formula (I) have also been found to be active against human tumour cells in primary cultures of lung, ovary, breast, renal, melanoma, unknown primary, gastric, pancreatic, mesothelioma, myeloma, and/or colon cancer. (As used herein "cancer" is to be taken as synonymous with "malignant tumour" or more generally "tumour" unless otherwise noted.) This is a procedure in which the prevention of tumour cell colony formation, i.e. tumour cell replication, by a drug has been shown to correlate with clinical antitumour activity in man (D.D. Von Hoff et al., Cancer Chemotherapy and Pharmacology 6, 265 (1980); S. Salmon and D.D. Von Hoff, Seminars in Oncology, 8, 377 (1981)).

Compounds of formula (I) which have been found to have antitumour activity intercalate in vitro with DNA (this property is determined by viscometric methods using the procedure of W. D. Wilson et al., Nucleic Acids Research 4, 2697 (1954)) and have a log P as calculated by the method of C. Hansch and A. Leo in Substituent Constants for Correlation Analysis in Chemistry and Biology, John Wiley and Sons, New York, 1979, lying in the range between -2.0 and +2.5.

As has been described above, the compounds of the present invention are useful for the treatment of tumours. The invention thus further provides a method for the treatment of tumours in animals, including mammals, especially humans, which comprises the administration of a clinically useful amount of compound of formula (I) in a pharmaceutically useful form, once or several times a day or other appropriate schedule, orally, rectally, parenterally, or applied topically.

In addition, there is provided as a further, or alternative, aspect of the invention, a compound of formula (I) for use in therapy, for example as an antitumour agent.

The amount of compound of formula (I) required to be effective as a biocidal agent will, of course, vary and is ultimately at the discretion of the medical or veterinary practitioner. The factors to be considered include the condition being treated, the route of administration, and nature of the formulation, the mammal's body weight, surface area, age and general condition, and the particular compound to be administered. A suitable effective antitumour dose is in the range of about 0.1 to about 120 mg/kg body weight, preferably in the range of about 1.5 to 50 mg/kg, for example 10 to 30 mg/kg. The total daily dose may be given as a single dose, multiple doses, e.g., two to six times per day or by intravenous infusion for selected duration. For example, for a 75 kg mammal, the dose range would be about 8 to 9000 mg per day, and a typical dose would be about 2000 mg per day. If discrete multiple doses are indicated, treatment might typically be 500 mg of a compound of formula I given 4 times per day in a pharmaceutically useful formulation.

Whilst it is possible for the active compound (defined herein as compound of formula (I), or ether, ester, or salt thereof) to be administered alone, it is preferable to present the active compound in a pharmaceutical formulation. Formulations of the present invention, for medical use, comprise an active compound together with one or more pharmaceutically acceptable carriers thereof and optionally other therapeutical ingredients. The carrier(s) must be pharmaceutically acceptable in the sense of being compatible with the other ingredients of the formulation and not deleterious to the recipient thereof.

The present invention, therefore, further provides a pharmaceutical formulation comprising a compound of formula (I) (in the form of the free base, ether, or ester derivative or a pharmaceutically acceptable acid addition salt thereof) together with a pharmaceutically acceptable carrier therefor.

There is also provided a method for the preparation of a pharmaceutical formulation comprising bringing into association a compound of formula (I) an ether, ester, or pharmaceutically acceptable salt thereof, and a pharmaceutically acceptable carrier therefor.

Whilst the antitumour activity of the compounds of formula (I) is believed to reside in the free base, it is often convenient to administer an acid addition salt of a compound of formula (I).

The formulations include those suitable for oral, rectal or parenteral (including subcutaneous, intramuscular and intravenous) administration. Preferred formulations are those suitable for oral or parenteral administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into assocation with a liquid carrier or a finely divided solid carrier or both and then, if necessary, shaping the product into desired formulations.

Formulations of the present invention suitable for oral administration may be presented as discrete units such as capsules, cachets, tablets or lozenges, each containing a predetermined amount of the active compound; as a powder or granules; or a suspension in an aqueous liquid or non-aqueous liquid such as a syrup, an elixir, an emulsion or a draught.

A tablet may be made by compression or moulding, optionally with one or more accessory ingredients. Compressed tablets may be prepared by compressing in a suitable machine the active compound in a free-flowing form such as a powder or granules, optionally mixed with a binder, lubricant, inert diluent, surface active or dispersing agent. Moulded tablets may be made by moulding in a suitable machine, a mixture of the powdered active compound with any suitable carrier.

A syrup may be made by adding the active compound to a concentrated, aqueous solution of a sugar, for example sucrose, to which may also be added any accessory ingredients. Such accessory ingredient(s) may include flavourings, an agent to retard crystallization of the sugar or an agent to increase the solubility of any other ingredient, such as a polyhydric alcohol for example glycerol or sorbitol.

Formulations for rectal administration may be presented as a suppository with a conventional carrier such as cocoa butter.

Formulations suitable for parenteral administration conveniently comprise a sterile aqueous preparation of the active compound which is preferably isotonic with the blood of the recipient. Such formulations suitably comprise a solution of a pharmaceutically and pharmacologically acceptable acid addition salt of a compound of the formula (I) that is isotonic with the blood of the recipient. Thus, such formulations may conveniently contain distilled water, 5% dextrose in distilled water or saline and a pharmaceutically and pharmacologically acceptable acid addition salt of a compound of the formula (I) that has an appropriate solubility in these solvents, for example the hydrochloride, isethionate and methanesulfonate salts, preferably the latter.

Useful formulations also comprise concentrated solutions or solids containing the compound of formula (I) which upon dilution with an appropriate solvent give a solution suitable for parenteral administration as above.

In addition to the aforementioned ingredients, the formulations of this invention may further include one or more accessory ingredient(s) selected from diluents, buffers, flavouring agents, binders, surface active agents, thickeners, lubricants, preservatives (including antioxidants) and the like.

The following examples are provided by the way of illustration of the present invention and should in no way be construed as a limitation thereof.

### General Comments

All solvents were reagent grade and used without further purification with the following exceptions. Tetrahydrofuran (THF) was dried by distillation from Na/K alloy under nitrogen (N₂) and used immediately. Toluene (PhCH₃) was distilled from CaH₂ under N₂ and stored over 3A molecular sieves. Chemicals used were reagent grade and used without purification unless noted. The full name and address of the suppliers of the reagents and chemicals is given when first cited. After this, an abbreviated name is used.

Preparative HPLC was carried out on a Waters Prep LC/System 500A machine using two 500 g silica gel (SiO₂) cartridges unless otherwise noted. Plugs of SiO₂ used for purifications were "flash chromatography" silica gel (Merck ^{·} Co., Inc., Merck Chemical Division, Rahway, NJ, 07065 silica gel 60, 230-400 mesh). An appropriate volume sintered glass funnel was filled approximately 3/4 full with the SiO₂ and packed evenly by tapping the outside of the funnel. A piece of filter paper was then placed on top of the SiO₂ and a solution of the material to be purified applied evenly to the top. Gentle suction through a filter flask moved the eluting solvent through the plug rapidly. The appropriate size fractions were combined as needed and further manipulated.

General procedures are described in detail. Analogous procedures show melting point (mp), recrystallization solvents, and elemental analyses (all elements analyzing within a difference of ±0.4% of the expected value). Any changes to the procedure such as solvent, reaction temperature, reaction time, or workup are noted.

NMR (¹H, ¹³C), IR, MS data of all new products were consistent with the expected and proposed structures. The positions assigned to structural isomers were unequivocally determined by a number of NMR techniques. All final products were dried in a vacuum oven at 20 mm Hg pressure at the temperature indicated overnight (12-16 hours) All temperatures are in degrees Celsius.

### EXAMPLE 1

### 2-[(1-Dibenzothiophenylmethyl)amino]-2-methyl-1,3-propanediol hydrochloride

To a round bottom flask equipped with magnetic stirring bar, condenser, thermometer, Dean-Stark trap, nitrogen inlet and bubbler was added dibenzothiophene-1-carbaldehyde (M.L. Tedjamulia et. al., J. Het. Chem. 21, 321 (1984), 10.0g, 65.7 mmol), 2-amino-2-methyl-1,3-propanediol (Aldrich, 6.91g, 65.7 mmol, p-toluenesulfonic acid monohydrate (Aldrich, 0.1g) and toluene (300 mL). The mixture was stirred at reflux with removal of water for 3h (or until no more water collects). Most of the toluene was then removed by distillation (200 mL). The mixture was then cooled in an ice bath and diluted with absolute EtOH (200 mL) and further cooled. Solid NaBH₄ (MCB Manufacturing Chemists, Inc., 2909 Highland Ave., Cincinnati, OH, 45212, 2.49g, 65.7 mmol) was added in one portion to the reaction mixture. The ice bath was then removed, the reaction allowed to warm to room temperature and then stirred overnight. The solvent was then removed from the reaction mixture by rotary evaporation to give a crude solid. This was shaken vigorously with warm H₂O (500 mL) and allowed to cool to room temperature, filtered and washed with additional H₂O and placed in a vacuum oven overnight (80°). The crude solid was transferred to flask and dissolved in a mixture of methanol and gaseous HCl dissolved in absolute EtOH filtered and diluted to 2L with diethyl ether. The white solid was recrystallized two additional times from methanol/diethyl ether (400 mL/600mL), filtered and washed with additional diethyl ether (300 mL) and placed in a vacuum oven at 80° overnight. A total of 7.38g (33.2% yield) of 2-[(1-dibenzothiophenyl-methyl)amino]-2-methyl-1,3-propanediol hydrochloride, mp 236-237° which analyzed correctly (C,H,N,Cl,S) for the assigned structure.

Other compounds which were isolated as their methanesulfonates were produced by treating the crude free base with methanesulfonic acid (99.5%, Morton Thiokol, Inc.-Alfa Products, PO Box 299, 152 Andover Street, Danvers, MA, 01923), followed by crystallization as described above. Other solvents such as absolute ethanol and isopropanol were used in combination with ethyl ether as the crystallization solvent.

### EXAMPLE 2

### 2-[(2-Dibenzothiophenylmethyl)amino]-2-methyl-1,3-propanediol hydrochloride

Using the procedure outlined in example 1, dibenzothiophene-2-carbaldehyde (E. Campaigne and J. Ashby, J. Het. Chem. 6,517 (1969)) and 2-amino-2-methyl-1,3-propanediol (Aldrich) gave 2-[(2-dibenzothiophenylmethyl)amino-2-methyl-1,3-propanediol hydrochloride, mp 194-194.5° in 33.1% yield which analyzed correctly (C,H,N,Cl,S) for the assigned structure.

### EXAMPLE 3

### 2-[(4-Dibenzothiophenylmethyl)amino]-2-methyl-1,3-propanediol hydrochloride

Using the procedure outlined in example 1, dibenzothiophene-4-carbaldehyde (M.L. Tedjamulia et. al., J. Het. Chem. 20, 86.1 (1983)) and 2-amino-2-methyl-1,3-propanediol (Aldrich) gave 2-[(4-dibenzothiophenylmethyl)amino]-2-methyl-1,3-propanediol hydrochloride, mp 194-194.5° in 33.1% yield which analyzed correctly (C,H,N,Cl,S) for the assigned structure.

### EXAMPLE 4

### 2-[(1-Dibenzofuranylmethyl)amino]-2-methyl-1,3-propanediol

### 4A Dibenzofuran-1-carbaldehyde ?******

### 4B 2-[(1-Dibenzofuranylmethyl)amino]-2-methyl-1,3-propanediol hydrochloride

Using the procedure outlined in example 1, dibenzofuran-1-carbaldehyde (4A) and 2-amino-2-methyl-1,3-propanediol (Aldrich) gave 2-[(1-dibenzofuranylmethyl)amino-2-methyl-1,3-propanediolhydrochloride, mp 237-238° in 41.4% yield which analyzed correctly (C,H,N,Cl) for the assigned structure.

### EXAMPLE 5

### 2-[(2-Dibenzofuranylmethyl)amino]-2-methyl-1,3-propanediol hydrochloride

Using the procedure outlined in example 1, dibenzofuran-2-carbaldehyde (J. Garmatter, A.E. Siegrist, Helv. Chim. Acta 57,945(1974)) and 2-amino-2-methyl-1,3-propanediol (Aldrich) gave 2-[(2-dibenzofuranylmethyl)amino]-2-methyl-1,3-propanediol hydrochloride, mp 237-238° in 41.4% yield which analyzed correctly (C,H,N,Cl) for the assigned structure.

### EXAMPLE 6

### 2-[(Naphtho[2,3-b]thiophen-4-ylmethyl)amino]-2-methyl-1,3-pronanediol

### 6A Naphtho[2,3-b]thiophen-4-carbaldehyde

Naphtho[2,3-b]-thiophene (H.G. Pars Pharmaceutical Laboratories, Inc.) was formylated using the procedure of A. Rieche et. al., Chem. Ber. 93,88 (1960) to give a mixture of crude naphtho[2,3-b]-thiophen-4-carbaldehyde and naphtho[2,3-b]thiophen-9-carbaldehyde in a 4:1 ratio respectively (by ¹H NMR) in 61.6% yield. The mixture could not be separated by chromatography or fractional crystallization. Reduction of the mixture with NaBH₄ in THF gave a mixture of the corresponding alcohols. After preparative HPLC (Waters HPLC 500A) using toluene as the eluting solvent and the shave/recycle technique, 4-hydroxymethylnaphtho[2,3-b]thiophene was obtained isomerically pure. This material was oxidized using BaMnO₄ to give after work up and crystallization (CH₂Cl₂/hexane) 2.95g (82.7%) of naphtho[2,3-b]thiophen-4-carbaldehyde, mp 113° which analyzed correctly (C,H,S) for the assigned structure.

### 6B 2-[(Naphtho[2,3-b]thiophen-4-ylmethyl)amino]-2-methyl-1,3-propanediol hydrochloride 1/20 hydrate

Using the procedure outlined in example 1, naphtho[2,3-b]thiophen-4-carbaldehyde (6A) and 2-methyl-2-amino-1,3-propanediol (Aldrich)) gave 2-[(naphtho[2,3-b]thiopen-4-ylmethyl)amino]-2-methyl-1,3-propanediol hydrochloride 1/20 hydrate, mp 205-207° (dec) in 56.6% yield which analyzed correctly (C,H,N,Cl,S) for the assigned structure.

### EXAMPLE 7

### 2-Methyl-2-[(naphtho[2,1-b]thiophen-5-ylmethyl)amino-1,3-propanediol

### 7A Methyl 5-formylnaphtho[2,1-b]thiophene-2-carboxylate

Methyl naphtho[2,1-b]thiophene-2-carboxylate (J. Org. Chem. 21, 39 (1956) was formylated (using the procedure of Riecha et. al., Chem. Ber 93, 88 (1960)) to give methyl 5-formylnaphtho-[2,1-b]thiophene-2-carboxylate, mp 196-198° in 52.4% yield which analyzed correctly (C,H,S) for the assigned structure.

### 7B Naphtho[2,1-b]thiophene-5-carbaldehyde

To a round bottom flask equipped with magnetic stirring bar, condenser and nitrogen inlet tube and bubbler was added methyl 5-formylnaphtho-[2,1-b]-thiophene-2-carboxylate (7A, 9.75g, 36 mmol), potassium hydroxide (85%, Mallinckrodt, 18.0g 320 mmol), CH₃OH (40 mL) and H₂O (80 mL). The mixture was refluxed for 1.5h, cooled and neutralized with 3N HCl (500 mL). A yellow solid formed which was filtered and washed with H₂O (3x 300 mL) and dried in a vacuum oven overnight to give crude 5-formylnaphtho-[2,1-b]-thiophene-2-carboxylic acid in quantitative yield. This material was then slurried with quinoline (Mallinckrodt, 60 mL) and cuprous oxide (MC and B, 6.2g, 43.3 mmol) and heated to 185° in an oil bath for 1h. The reaction mixture was allowed to cool to room temperature then partitioned between diethyl ether (500 mL) and concentrated HCl (200 mL) containing saturated NH₄Cl (100 mL). The layers were separated and the aqueous layer extracted with additional diethyl ether (500 mL). The ether layers were combined and washed in succession with 1N HCl (300 mL), H₂O (2x 300 mL) and saturated NaCl solution (2x 300 mL), dried (MgSO₄), filtered and concentrated to give a light brown crude solid. After chromatography on silica gel using CHCl₃/hexane as the eluting solvent and crystallization (CHCl₃/cyclohexane) there was obtained 4.98g (65.2% yield) of naphtho[2,1-b]thiophene-5-carbaldehyde, mp 101-102° which analyzed correctly (C,H,S) for the assigned structure.

### 7C 2-Methyl-2-[(naphtho[2,1-b]thiophen-5-ylmethyl)amino]-1,3-propanediol hydrochloride

Using the procedure outlined in example 1, naphtho[2,1-b]-thiophene-5-carbaldehyde (7B) and 2-methyl-2-amino-1,3-propanediol (Aldrich) gave 2-methyl-2-[(naphtho[2,1-b]-thiophen-5-ylmethyl)amino]-1,3-propanediol hydrochloride, mp 208.5-210° (dec) in 80.9% yield which analyzed correctly (C,H,N,Cl,S) for the assigned structure.

### EXAMPLE 8

### 2-Methyl-2-[(naphtho[2,1-b]thiophen-2-ylmethyl)amino]-1,3-propanediol hydrochloride

Using the procedure outlined in example 1, naphtho[2,1-b]-thiophene-2-carbaldehyde (K. Clarke et. al., J. Chem. Soc. C. 537 (1969)) and 2-methyl-2-amino-1,3-propanediol (Aldrich) gave 2-methyl-2-[(naphtho[2,1-b]thiophen-2-ylmethyl)amino]-1,3-propanediol hydrochloride, mp 189-190° in 75.2% yield which analyzed correctly (C,H,N,Cl,S) for the assigned structure.

### EXAMPLE 9

### 2-Methyl-2-[(naphtho[1,2-b]thiophen-2-ylmethyl)amino)-1,3-propanediol hydrochloride

Using the procedure outlined in example 1, naphtho[1,2-b]-thiophene-2-carbaldehyde (M.L. Tedjamulia et. al., J. Het. Chem. 20, 1143 (1983)) and 2-methyl-2-amino-1,3-propanediol (Aldrich) gave 2-methyl-2-[(naphtho[1,2-b]-thiophen-2-yl-methyl)amino]-1,3-propanediol hydrochloride, mp 224-224.5° in 53.3% yield which analyzed correctly (C,H,N,Cl,S) for the assigned structure.

### EXAMPLE 10

### 2-Methyl-2-[(naphtho[1,2-b]thiophen-5-ylmethyl)amino]-1,3-propanediol

### 10A Methyl 5-formyl-naphtho[1,2-b]thiophene-2-carboxylate

Using the procedure outlined in example 7A, methyl naphtho[1,2-b]-thiophene-2-carboxylate (J. Org. Chem. 21, 39 (1956) gave methyl 5-formylnaphtho[1,2-b]thiophene-2-carboxylate, mp 207.5-208.5° (61% yield) which analyzed correctly (C,H,S) for the assigned structure.

### 10B Naphtho[1,2-b]thiophene-5-carbaldehyde

Using the procedure outlined in 7B, methyl naphtho[1,2-b]-thiophene-2-carboxylate (10A) gave naphtho[1,2-b]thiophene-5-carbaldehyde, mp 96-96.5° in 62% yield which analyzed correctly (C,H,S) for the assigned structure.

### 10C 2-Methyl-2-[(naphtho[1,2-b]thiophene-5-ylmethyl)amino]-1,3-propanediol

Using the procedure outlined in example 1, naphtho[1,2-b]-thiophene-5-carbaldehyde (10B) and 2-methyl-2-amino-1,3-propanediol (Aldrich) gave 2-methyl-2-[(naphtho[1,2-b]-thiophen-5-ylmethyl)amino]-1,3-propanediol hydrochloride, mp 196-198° (dec) in 61.3% yield which analyzed correctly (C,H,N,Cl,S) for the assigned structure.

### EXAMPLE 11

### 2-Methyl-2-[(naphtho[1,2-b]furan-2-ylmethyl)amino]-1,3-propanediol

### 11A Naphtho[1,2-b]furan-2-methanol

To a round bottom flask equipped with magnetic stirring bar, reflux condenser, nitrogen inlet tube and bubbler was added ethyl naphtho[1,2-b]furan-2-carboxylate (H.G. Pars Pharmaceutical Laboratories, Inc., 6.85g, 28.5 mmol), lithium borohydride (Aldrich, 0.62g, 28.5 mmol) and dry THF (400 mL). The mixture was stirred at reflux for 6h and then poured into H₂O (1L). The reaction mixture was acidified with 1N HCl and the resulting white solid was filtered, washed with additional H₂O (500 mL) then dissolved in CH₂Cl₂ (500 mL), dried (Na₂SO₄), filtered, concentrated to 200 mL and diluted to 500 mL with hexane.

The resulting material was filtered, washed with hexane (100 mL) and placed in a vacuum oven overnight. A total of 4.8g of naphtho[1,2-b]furan-2-methanol, mp 105.5-107° was obtained which analyzed correctly (C,H) for the assigned structure.

### 11B Naphtho[1,2-b]furan-2-carbaldehyde

To a round flask equipped with magnetic stirring bar, reflux condenser, nitrogen inlet tube and bubbler was added naphtho[1,2-b]-furan-2-methanol (11A, 4.8g, 24.2 mmol), barium manganate (Aldrich, 12.4g, 48 mmol) and dry CH₂Cl₂ (500 mL). The mixture was refluxed for 6h, filtered and the resulting dark yellow solution filtered through a small plug of silica gel to remove inorganic salts and baseline material. The solvent was then removed by rotary evaporation and the crude material recrystallized using CH₂Cl₂/ pentane to give after drying 4.02g (84.7% yield) of phenanthro[1,2-b]furan-2-carbaldehyde, mp 123° which analyzed correctly (C,H) for the assigned structure.

### 11C 2-Methyl-2-[(naphtho[1,2-b]furan-2-ylmethyl)amino]-1,3-propanediol hydrochloride

Using the procedure outlined in example 1, naphtho[1,2-b]furan-2-carbaldehyde (11B) and 2-methyl-2-amino-1,3-propanediol (Aldrich) gave 2-methyl-2-[(naphtho[1,2-b]furan-2-ylmethyl)amino]-1,3-propanediol hydrochloride, mp 197-199° (dec) in 43.4% yield which analyzed correctly (C,H,N,Cl) for the assigned structure

### EXAMPLE 12

### 2-Methyl-2-[(naphtho[2,1-b]furan-2-ylmethyl)amino]-1,3-propanediol hydrochloride

Using the procedure outlined in example 1, naphtho[2,1-b]furan-2-carbaldehyde (G. Giovaninetti et. al., Farmaco, Ed. Sci. 36, 94 (1981) and 2-methyl-2-amino-1,3-propanediol (Aldrich) gave 2-methyl-2-[(naphtho[2,1-b]furan-2-ylmethyl)amino]-1,3-propanediol hydrochloride, mp 228-230° (dec) in 51.1% yield which analyzed correctly (C,H,N,Cl,) for the assigned structure.

### EXAMPLE 13

### 2-[(9-Acridinylmethyl)amino]-2-methyl-1,3-propanediol dihydochoride

Bromination of 9-methylacridine (Lancaster Synthesis Ltd., P.O. Box 1000, Industrial Drive, Wyndham, NH 03087) by the procedure of A. Campbell et al, J. Chem. Soc. 1145 (1958) gave 9-bromomethylacridine. To a round bottomed flask equipped with magnetic stirring bar, condenser, and nitrogen inlet tube and bubbler was added 9-bromomethylacridine (10.5g, 38.58 mmol), 2-amino-2-methyl-1,3-propanediol (Aldrich Chemical Co., Milwaukee, WI, 53201, 4.06g, 38.58 mmol), anhydrons potassium carbonate (Mallinckrodt Co., St. Louis, MO, 53147, 10.50g, 76.0 mmol) and abs. ethanol (150 mL). The mixture was refluxed for 4 hours, cooled and filtered. The solvent was then removed by rotary evaporation. The crude product was dissolved in methanol (200 mL) filtered again and diluted to 2L with diethyl ether. The dark coloured crude product wasthen recrystallized one additional time from methanol+/diethyl ether and then twice from methanol to give 2-[(9-Acridinylmethyl)amino]-2-methyl-1,3-propanediol 0.6H₂O mp 210-211°(d), which analysed correctly for the assigned strucure (C,H,N,Cl).

### EXAMPLE 14

### 2-[(Acenaphtho[1.2-b]quinolin-10-ylmethyl)amino]-2-methyl-1,3-propanediol

### 14A Acenaphtho[1,2-b]quinoline-10-methanol

To a round bottom flask equipped with magnetic stirring bar, reflux condenser, nitrogen inlet tube and bubbler was added ethyl acenaphtho[1,2-b]quinoline-10-carboxylate (H.G. Pars Pharmaceutical Laboratories, Inc. 6.75g, 23 mmol), lithium borohydride (Aldrich, 0.5g, 23 mmol) and dry THF (400 mL). The mixture was stirred at reflux for 3h and then poured into H₂O (1L). The reaction mixture was acidified with 1N HCl and the resulting white solid was filtered, washed with additional H₂O (500 mL) then dissolved in CH₂Cl₂ (500 mL), dried (Na₂SO₄), filtered, passed through a small plug of silica gel using CH₂Cl₂ as the eluting solvent. The appropriate fractions were combined and the volume reduced to 100 mL and diluted to 400 mL with hexane. The resulting material was filtered, washed with hexane (100 mL) and placed in a vacuum oven overnight. A total of 5.52g of acenaphthol[1,2-b]quinoline-10-methanol, mp 215-218° was obtained which analyzed correctly (C,H,N) for the assigned structure.

### 14B Acenaphtho[1,2-b]quinoline-10-carbaldehyde

To a round bottom flask equipped with magnetic stirring bar, reflux condenser, nitrogen inlet tube and bubbler was added acenaphtho-[1,2-b]quinoline-10-methanol (14A, 2.25g, 8 mmol), barium manganate (Aldrich, 40g, 16 mmol) and dry CH₂Cl₂ (1L). The mixture was refluxed for 1 day, filtered and the resulting dark yellow solution filtered and the solvent removed by rotary evaporation to give a dark green solid. This material was dissolved in a mixture of EtOAc (700 mL) and THF (200 mL) and passed through a small plug of silica gel and eluted using EtOAc as the eluting solvent. The appropriate fractions were combined and the solvent removed by rotary evaporation to give acenaphtho[1,2-b]quinoline-10-carbaldehyde, mp 245-246° in 71.1% yield which analyzed correctly (C,H,N) for the assigned structure.

### 14C 2-[(Acenaphtho[1,2-b]quinolin-10-ylmethyl)amino]-2-methyl-1,3-propanediol Dihydrochloride 1.25 Hydrate

Using the procedure outlined in example 1, acenaphtho[1,2-b]quinoline-10-carbaldehyde (14B) and 2-methyl-2-amino-1,3-propanediol (Aldrich) gave 2-[ (acenaphtho[1,2-b]quinolin-10-yl-methyl)amino]-2-methyl-1,3-propanediol dihydrochloride 1.25 hydrate, mp 242-245° (dec) in 55.7% yield which analyzed correctly (C,H,N,Cl,) for the assigned structure.

### EXAMPLE 15

### 2-[Acenaphtho-[1,2-b]-thiophen-8-ylmethyl)amino]-2-methyl-1,3-propanediol hydrochloride

Using the procedure outlined in example 1, acenaphtho[1,2-b]thiophene-8-carbaldehyde (H.G. Pars Pharmaceutical Laboratories, Inc.) and 2-amino-2-methyl-1,3-propanediol (Aldrich) gave 2-[acenaphtho-[1,2-b]thiophen-8-ylmethyl)amino]-2-methyl-1,3-propanediol hydrochloride, mp 208-210° (dec) in 73.8% yield which analyzed correctly (C,H,N,Cl,S) for the assigned structure.

### EXAMPLE 16

### 2-[Acenaphtho-[1,2-c]-thiophen-7-ylmethyl)amino)-2-methyl-1,3-propanediol hydrochloride

### 16A Acenaphtho[1,2-c]-thiophene-7-carbaldehyde

Using the procedure outlined in example 2, acenaphtho[1,2-c]-thiophene (H.G. Pars Pharmaceutical Laboratories, Inc.) gave acenaphtho[1,2-c]-thiophene-7-carbaldehyde, mp 123-125.5° in 83.2% yield which analyzed correctly (C,H,S) for the assigned structure.

### 16B 2-[Acenaphtho[1,2-c]-thiophen-7-ylmethyl)amino]-2-methyl-1,3-propanediol hydrochloride

Using the procedure outlined in example 1, acenophtho[1,2-c]-thiophene-7-carbaldehyde (16A) and 2-amino-2-methyl-1,3-propanediol (Aldrich) gave 2-[acenaphtho-[1,2-c]-thiophen-7-ylmethyl)amino]-2-methyl-1,3-propanediol hydrochloride, mp 220-223° (dec) in 57.4% yield which analyzed correctly (C,H,N,Cl,S) for the assigned structure.

### Antitumor Screening Results

Methods for evaluating the antitumor activity of these compounds are essentially those used in the Tumor Panel by the Development Therapeutics Program, Division of Cancer Treatment, National Cancer Institute, A. Goldin, et al., Methods in Cancer Research, Vol. XVI, p. 165, Academic Press (1979). Some modifications, in dose level and schedule have been made to increase the testing efficiency.

### Lymphocytic Leukemia P388/0 Test

CD2-F₁ mice, of the same sex, weighing within a 3 g range surrounding 20 g, are used for this test. Control and test animals are injected intraperitoneally with a suspension of 10⁶ viable P388/0 tumor cells on day 0. In each test several dose levels which bracket the LD₂₀ for the compound are evaluated; each dose level group contains 6 animals. The test compounds are prepared either in physiologic saline containing 0.05% Tween 80 or distilled water containing 5% dextrose and are administered intraperitoneally on days, 1, 5, and 9 relative to tumor implant.

Doses are on a mg/kg basis according to individual animals' body weights. The day of death for each animal is recorded, the median identified for each group and the ratios of median survival time for treated (T)/control (C) groups are calculated. The criterion for activity is T/C x 100 120%. Results of P388/0 testing are summarized in Table I below.

**TABLE I**

| compound of Formula | Optimal Dose (mg/kg) | T/C x 100% (Exluding 30 Day Survivors) | LD₂₀ (mg/kg) |
|---|---|---|---|
| 13 | 440 | +130 | 540 |
| 1 | 240 | +140 | 240 |
| 2 | 225 | +130 | 200 |
| 3 | 275 | +155 | 200 |
| 4B | 225 | +140 | 200 |
| 5 | 225 | +120 | 225 |
| 6B | 150 | +125 | 150 |
| 10C | 150 | +125 | 115 |

### Formulation Examples

| A. TABLET | |
|---|---|
| Compound of Formula I (as hydrochloride) | 500.0. mg |
| Pregelatinised Corn Starch | 60.0 mg |
| Sodium Starch Glycolate | 36.0 mg |
| Magnesium Stearate | 4.0 mg |

The Compound of formula (I) is finely ground and intimately mixed with the powdered excipients, pregelatinised corn starch and sodium starch glycolate. The powders are wetted with purified water to form granules. The granules are dried and mixed with the magnesium stearate. The formulation is then compressed into tablets weighing approximately 600 mg each.

| B. TABLET | |
|---|---|
| Compound of formula (I) | 500.0 mg |
| Corn Starch | 70.0 mg |
| Lactose | 83.8 mg |
| Magnesium Stearate | 4.2 mg |
| Polyvinylpyrrolidone | 14.0 mg |
| Stearic Acid | 28.0 mg |

The Compound of formula (I) is finely ground and intimately mixed with the powdered excipients, corn starch and lactose. The powders are wetted with a solution of polyvinylpyrrolidone dissolved in purified water and denatured alcohol to form granules. The granules are dried and mixed with the powdered stearic acid and magnesium stearate. The formulation is then compressed into tablets weighing approximately 700 mg each.

| C. CAPSULES | |
|---|---|
| Compound of formula (I) | 500.0 mg |
| Corn Starch | 50.0 mg |
| Magnesium Stearate | 3.0 mg |

The finely divided compound of formula (I) is mixed with powdered corn starch and wetted with denatured alcohol to densify the powder. The dried powder is mixed with stearic acid and filled into hard-shell gelatin capsules.

| D. SYRUP | |
|---|---|
| Compound of formula (I) | 250.0 mg |
| Ethanol | 250.0 mg |
| Glycerin | 500.0 mg |
| Sucrose | 3,500.0 mg |
| Flavouring Agent | q.s. |
| Colouring Agent | q.s. |
| Presserving Agent | 0.1% |
| Purified Water | q.s.to 5.0 ml |

The Compound of formula (I) is dissolved in the ethanol, glycerin, and a portion of the purified water. The sucrose and preserving agent are dissolved in another portion of hot purified water, and then the colouring agent is added and dissolved. The two solution are mixed and cooled before the flavouring agent is added. Purified water is added to final volume. The resulting syrup is throughly mixed.

| E. IV INJECTION | |
|---|---|
| Compound of formula (I) (as methanesulphonate) | 5.0 mg |
| Glycerin | q.s. for isotonicity |
| Presservative | 0.1% |
| Hydrochloric Acid or | as needed for |
| Sodium Hydroxide | pH adjustment |
| Water for Injection | q.s. to 1 ml |

The compound of formula (I) and preservative is added to the glycerin and a portion of the water for injection. The pH is adjusted with hydrochloric acid or sodium hydroxide. Water for injection is added to final volume and solution is complete after thorough mixing. The solution is sterilised by filtration through a 0.22 micrometer membrane filter and aseptically filled into sterile 10 ml ampoules or vials.

## Claims (Claims for the following Contracting State(s): BE, CH, DE, FR, GB, IT, LI, LU, NL, SE)

1. A compound of the of the formula (I)
**RCH₂NHR¹**
or a monomethyl or monoethyl ether therof, the compound of formula (I) including its ethers containing no more than 29 carbon atoms in total, or on ester or a salt thereof;
Wherein R is a fused ring system containing 3, 4 or 5 rings and a maximum of 4n+1 carbon atoms , n being the number of rings present, at least three rings being aromatic when n is 3 or 4 and at least four rings being aromatic when n is 5, at least one of the aromatic rings containing a heteroatom; optionally substituted by one or two substituents; said substituents containing not more than four carbon atoms in total when taken together and being the same or different and are selected from halogen; cyano; C₁₋₄ alkyl or C₁₋₄ alkoxy, each optionally substituted by hydroxy or C₁₋₂ alkoxy; halogen substituted C₁₋₂ alkyl or C₁₋₂ alkoxy; a group S(O)_{n'}R² wherein n' is an integer o, 1 or 2 and R² is C₁₋₂ alkyl optionally substituted by hydroxy or C₁₋₂ alkoxy, or R is optionally substituted by a group NR³R⁴ containing not more than 5 carbon atoms wherein R³ and R⁴ are the same or different and each is a C₁₋₃ alkyl group or NR³R⁴ forms a five or six-membered heterocyclic ring optionally containing one or two additional heteroatoms;
R¹ is 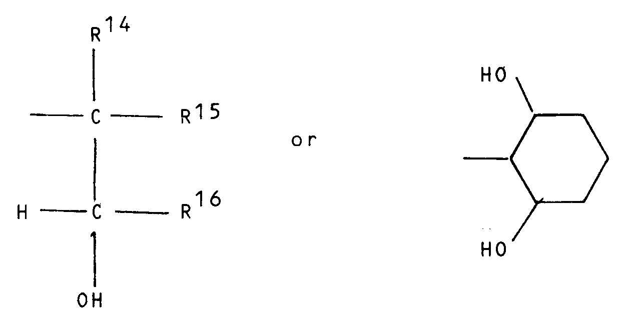 Wherein
R¹⁴ is CH₂OH, CH(CH₃)OH or CH₂CH₂OH
R¹⁵ is hydrogen, C₁₋₃ alkyl or CH₂OH
R¹⁶ is hydrogen or methyl
Preferably R¹⁴ is CH₂OH or CH(CH₃)OH; preferably R¹⁵ is hydrogen, methyl, ethyl or CH₂OH; except that R is not a tetracyclic ring system containing 15 to 17 ring atoms, three six membered carbocyclic rings and one five membered ring in which there is a heteroatom.

2. A compound according to claim 1 wherein only one or two of the aromatic rings contains or contain a heteroatom.

3. A compound according to either of claims 1 or 2 wherein the heteroatom or heteroatoms is or are selected from nitrogen, phosphorus, oxygen, sulphur, or selenium.

4. A compound according to claim 3 wherein the heteroatom or heteroatoms is or are selected from oxygen, nitrogen or sulphur.

## Claims (Claims for the following Contracting State(s): AT)

1. A process for the preparation of a compound of the formula (I)
RCH₂NHR¹
or a monomethyl ether thereof, the compound of formula (I) including its ethers containing no more than 29 carbon atoms in total, or an ester or a salt thereof;
wherein R is a fused ring system containing 3, 4 or 5 rings and a maximum of 4n+1 carbon atoms, n being the number of rings present, at least three rings being aromatic when n is 3 or 4 and at least four rings being aromatic when n is 5, at least one of the aromatic rings containing a heteroatom; optionally substituted by one or two substituents; said substituents containing not more than four carbon atoms in total when taken together and being the same or different and are selected from halogen; cyano; C₁₋₄ alkyl or C₁₋₄ alkoxy, each optionally substituted by hydroxy or C₁₋₂ alkoxy; halogen substituted C₁₋₂ alkyl or C₁₋₂ alkoxy; a group S(O)_{n'}R² wherein n' is an integer O, 1 and 2 and R² is C₁₋₂ alkyl optionally substituted by hydroxy or C₁₋₂ alkoxy; or R is optionally substituted by a group NR³R⁴ containing not more than 5 carbon atoms wherein R³ and R⁴ are the same or different and each is a C₁₋₃ alkyl group or NR³R⁴ forms a five-or six-membered heterocyclic ring optionally containing one or two additional heteroatoms;
R¹ 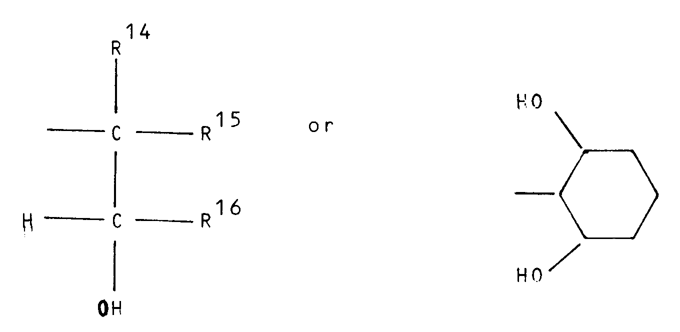 wherein
R¹⁴ is CH₂OH, CH(CH₃)OH or CH₂CH₂OH
R¹⁵ is hydrogen, C₁₋₃ alkyl or CH₂OH
R¹⁶ is hydrogen or methyl
Preferably R¹⁴ is CH₂OH or CH(CH₃)OH; preferably R¹⁵ is hydrogen, methyl, ethyl, or CH₂OH; except that R is not a tetracyclic ring system containing 15 to 17 ring atoms, three six membered carbocyclic rings and one five membered carbocyclic ring in which there is a heteroatom, which process comprises:
(a) the reduction of a compound R-CH=N-R¹ or an appropriately protected derivative thereof, followed by deprotection where appropriate;
(b) the reduction of a compound R.CO.NHR¹ wherein the hydroxy groups are optionally protected, followed by deprotection where appropriate; and
(c) the reaction of a compound RCH₂L with a compound NH₂R¹, wherein R and R¹ are as hereinbefore defined and L is a leaving group.

2. A method according to claim 1 for the preparation of a compound of the formula (I) wherein only one or two of the aromatic rings contains or contains a heteroatom.

3. A method according to either of claims 1 or 2 for the preparation of a compound of the formula (I) wherein the heteroatom or heteroatoms is or selected from nitrogen, phosphorus, oxygen, sulphur or selenium.

4. A method according to claim 3 for the preparation of a compound of the formula (I) wherein the heteroatom or heteroatoms is or are selected from oxygen, nitrogen, or sulphur.

5. A method according to claim 1 for the preparation of a compound wherein the fused ring system is of the formula:

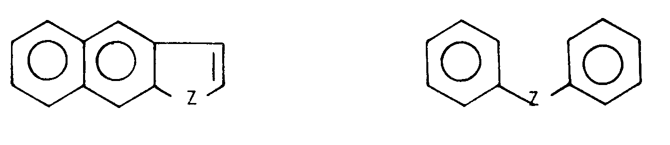

## Patentansprüche (Patentansprüche für folgende(n) Vertragsstaat(en): BE, CH, DE, FR, GB, IT, LI, LU, NL, SE)

1. Verbindung der Formel (I)
RCH₂NHR¹
oder ein Monomethyl- oder Monoethylether davon, wobei die Verbindung der Formel (I) einschließlich ihrer Ether nicht mehr als 29 Kohlenstoffatome enthält, oder ein Ester oder ein Salz davon;
worin R ein kondensiertes Ringsystem ist, welches 3, 4 oder 5 Ringe und maximal 4n+1 Kohlenstoffatome umfaßt, worin n für die Anzahl der vorhandenen Ringe steht, mindestens drei Ringe aromatisch sind, wenn n 3 oder 4 ist, und mindestens vier Ringe aromatisch sind, wenn n 5 bedeutet, und mindestens einer der aromatischen Ringe ein Heteroatom enthält; welches gegebenenfalls durch einen oder zwei Substituenten substituiert ist; welche Substituenten zusammengenommen insgesamt nicht mehr als 4 Kohlenstoffatome enthalten und gleichartig oder verschieden sind und ausgewählt sind aus Halogenatomen; Cyanogruppen; C₁₋₄-Alkylgruppen oder C₁₋₄-Alkoxygruppen, die jeweils gegebenenfalls durch Hydroxygruppen oder C₁₋₂-Alkoxygruppen substituiert sind; halogensubstituierten C₁₋₂-Alkylgruppen oder C₁₋₂-Alkoxygruppen; und Gruppen S(O)_{n'}R², worin n' eine ganze Zahl mit einem Wert von 0, 1 oder 2 und R² eine gegebenenfalls durch Hydroxygruppen oder C₁₋₂-Alkoxygruppen substituierte C₁₋₂-Alkylgruppe darstellen; oder R gegebenenfalls durch eine Gruppe NR³R⁴ substituiert ist, die nicht mehr als 5 Kohlenstoffatome aufweist und worin R³ und R⁴ gleichartig oder verschieden sind und jeweils C₁₋₃-Alkylgruppen darstellen, oder NR³R⁴ einen fünfgliedrigen oder sechsgliedrigen heterocyclischen Ring, der gegebenenfalls ein oder zwei zusätzliche Heteroatome enthält, bildet; und
R¹ 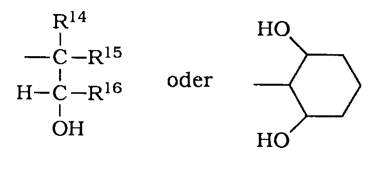 bedeutet,
worin
R¹⁴ CH₂OH, CH(CH₃)OH oder CH₂CH₂OH,
R¹⁵ Wasserstoff, eine C₁₋₃-Alkylgruppe oder CH₂OH und
R¹⁶ Wasserstoff oder eine Methylgruppe
bedeuten,
wobei vorzugsweise R¹⁴ CH₂OH oder CH(CH₃)OH bedeutet; vorzugsweise R¹⁵ ein Wasserstoffatom, eine Methylgruppe, eine Ethylgruppe oder CH₂OH darstellt; mit der Maßgabe, daß R kein tetracyclisches Ringsystem ist, welches 15 bis 17 Ringatome, drei sechsgliedrige carbocyclische Ringe und einen fünfgliedrigen Ring mit einem Heteroatom umfaßt.

2. Verbindung nach Anspruch 1, worin lediglich einer oder zwei der aromatischen Ringe ein Heteroatom enthält bzw. enthalten.

3. Verbindung nach den Ansprüchen 1 oder 2, worin das oder die Heteroatome ausgewählt sind aus Stickstoff, Phosphor, Sauerstoff, Schwefel und Selen.

4. Verbindung nach Anspruch 3, worin das Heteroatom oder die Heteroatome ausgewählt sind aus Sauerstoff, Stickstoff und Schwefel.

## Patentansprüche (Patentansprüche für folgende(n) Vertragsstaat(en): AT)

1. Verfahren zur Herstellung einer Verbindung der Formel (I)
RCH₂NHR¹
oder eines Monomethyl- oder Monoethylethers davon, wobei die Verbindung der Formel (I) einschließlich ihrer Ether nicht mehr als 29 Kohlenstoffatome enthält, oder eines Esters oder eines Salzes davon;
worin R ein kondensiertes Ringsystem ist, welches 3, 4 oder 5 Ringe und maximal 4n+1 Kohlenstoffatome umfaßt, worin n für die Anzahl der vorhandenen Ringe steht, mindestens drei Ringe aromatisch sind, wenn n 3 oder 4 ist, und mindestens vier Ringe aromatisch sind, wenn n 5 bedeutet, und mindestens einer der aromatischen Ringe ein Heteroatom enthält; welches gegebenenfalls durch einen oder zwei Substituenten substituiert ist; welche Substituenten zusammengenommen insgesamt nicht mehr als 4 Kohlenstoffatome enthalten und gleichartig oder verschieden sind und ausgewählt sind aus Halogenatomen; Cyanogruppen; C₁₋₄-Alkylgruppen oder C₁₋₄-Alkoxygruppen, die jeweils gegebenenfalls durch Hydroxygruppen oder C₁₋₂-Alkoxygruppen substituiert sind; halogensubstituierten C₁₋₂-Alkylgruppen oder C₁₋₂-Alkoxygruppen; und Gruppen S(O)_{n'}R², worin n' eine ganze Zahl mit einem Wert von 0, 1 oder 2 und R² eine gegebenenfalls durch Hydroxygruppen oder C₁₋₂-Alkoxygruppen substituierte C₁₋₂-Alkylgruppe darstellen; oder R gegebenenfalls durch eine Gruppe NR³R⁴ substituiert ist, die nicht mehr als 5 Kohlenstoffatome aufweist und worin R³ und R⁴ gleichartig oder verschieden sind und jeweils C₁₋₃-Alkylgruppen darstellen, oder NR³R⁴ einen fünfgliedrigen oder sechsgliedrigen heterocyclischen Ring, der gegebenenfalls ein oder zwei zusätzliche Heteroatome enthält, bildet; und
R¹ 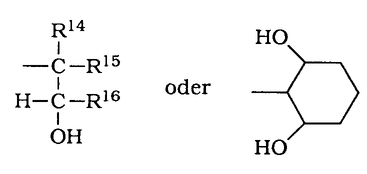 bedeutet,
worin
R¹⁴ CH₂OH, CH(CH₃)OH oder CH₂CH₂OH,
R¹⁵ Wasserstoff, eine C₁₋₃-Alkylgruppe oder CH₂OH und
R¹⁶ Wasserstoff oder eine Methylgruppe
bedeuten,
wobei vorzugsweise R¹⁴ CH₂OH oder CH(CH₃)OH bedeutet; vorzugsweise R¹⁵ ein Wasserstoffatom, eine Methylgruppe, eine Ethylgruppe oder CH₂OH darstellt; mit der Maßgabe, daß R kein tetracyclisches Ringsystem ist, welches 15 bis 17 Ringatome, drei sechsgliedrige carbocyclische Ringe und einen fünfgliedrigen Ring mit einem Heteroatom umfaßt, welches Verfahren gekennzeichnet ist durch:
(a) die Reduktion einer Verbindung R-CH=N-R¹ oder eines in geeigneter Weise geschützten Derivats davon, gefolgt von der Abspaltung der Schutzgruppe, falls erforderlich;
(b) die Reduktion einer Verbindung R.CO.NHR¹, worin die Hydroxygruppen gegebenenfalls geschützt sind, gefolgt von einer Abspaltung der Schutzgruppen, falls erforderlich; und
(c) die Umsetzung einer Verbindung RCH₂L mit einer Verbindung NH₂R¹, worin R und R¹ die oben angegebenen Bedeutungen besitzen und L eine austretende Gruppe darstellt.

2. Verfahren nach Anspruch 1 zur Herstellung einer Verbindung der Formel (I), worin lediglich einer oder zwei der aromatischen Ringe ein Heteroatom enthält bzw. enthalten.

3. Verfahren nach einem der Ansprüche 1 oder 2 zur Herstellung einer Verbindung der Formel (I), worin das Heteroatom oder die Heteroatome ausgewählt sind aus Stickstoff, Phosphor, Sauerstoff, Schwefel und Selen.

4. Verfahren nach Anspruch 3 zur Herstellung einer Verbindung der Formel (I), worin das Heteroatom oder die Heteroatome ausgewählt sind aus Sauerstoff, Stickstoff und Schwefel.

5. Verfahren nach Anspruch 1 zur Herstellung einer Verbindung, deren kondensiertes Ringsystem der Formel entspricht:

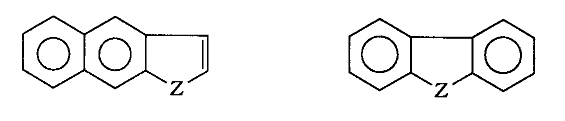

## Revendications (Revendications pour l'(les) Etat(s) contractant(s) suivant(s): BE, CH, DE, FR, GB, IT, LI, LU, NL, SE)

1. Composé de formule (I)
RCH₂NHR¹ (I)
ou un éther monométhylique ou monoéthylique de celui-ci, le composé de formule (I), y compris ses éthers, ne comptant pas plus de 29 atomes de carbone au total, ou un ester ou sel de celui-ci,
où R est un système cyclique condensé contenant 3, 4 ou 5 cycles et un maximum de 4n+1 atomes de carbone, n étant le nombre de cycles présents, au moins trois cycles étant aromatiques lorsque n est 3 ou 4 et au moins quatre cycles étant aromatiques lorsque n est 5, au moins l'un des cycles aromatiques contenant un hétéroatome; facultativement substitué par un ou deux substituants; ces substituants ne contenant pas plus de quatre atomes de carbone au total, lorsqu'ils sont pris ensemble, et étant identiques ou différents et étant choisis parmi halogène; cyano; C₁₋₄alcoyle et C₁₋₄alcoxy, chacun facultativement substitué par hydroxyle ou C₁₋₂alcoxy; C₁₋₂alcoyle ou C₁₋₂alcoxy halogéno-substitué; un radical S(O)_{n'}R², où n' est un entier, 0, 1 ou 2 et R² est C₁₋₂alcoyle facultativement substitué par hydroxyle ou C₁₋₂alcoxy; ou R est facultativement substitué par un radical NR³R⁴ ne contenant pas plus de 5 atomes de carbone, où R³ et R⁴ sont identiques ou différents et sont chacun un radical C₁₋₃alcoyle ou NR³R⁴ forme un cycle hétérocyclique de cinq ou six chaînons contenant facultativement un ou deux hétéroatomes supplémentaires;
où R¹ est 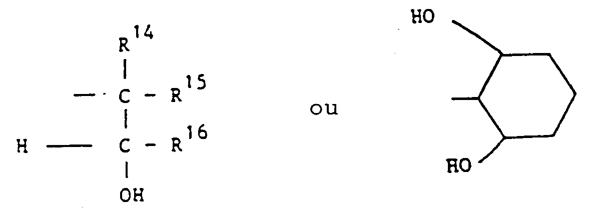 ou
R¹⁴ est CH₂OH, CH(CH₃)OH ou CH₂CH₂OH,
R¹⁵ est hydrogène, C₁₋₃alcoyle ou CH₂OH,
R¹⁶ est hydrogène ou méthyle;
de préférence, R¹⁴ est CH₂OH ou CH(CH₃)OH; de préférence, R¹⁵ est hydrogène, méthyle, éthyle ou CH₂OH, sauf que R n'est pas un système cyclique tétracyclique contenant 15 à 17 atomes de carbone, trois cycles carbocycliques de six chaînons et un cycle de cinq chaînons dans lequel il y a un hétéroatome.

2. Composé suivant la revendication 1, dans lequel un seul ou deux des cycles aromatiques contiennent un hétéroatome.

3. Composé suivant la revendication 1 ou 2, dans lequel le ou les hétéroatomes sont choisis entre l'azote, le phosphore, l'oxygène, le soufre et le sélénium.

4. Composé suivant la revendication 3, dans lequel le ou les hétéroatomes sont choisis entre l'oxygène, l'azote et le soufre.

## Revendications (Revendications pour l'(les) Etat(s) contractant(s) suivant(s): AT)

1. Procédé de préparation d'un composé de formule (I)
RCH₂NHR¹ (I)
ou d'un éther monométhylique de celui-ci, le composé de formule (I), y compris ses éthers, ne comptant pas plus de 29 atomes de carbone au total, ou d'un ester ou sel de celui-ci,
où R est un système cyclique condensé contenant 3, 4 ou 5 cycles et un maximum de 4n+1 atomes de carbone, n étant le nombre de cycles présents, au moins trois cycles étant aromatiques lorsque n est 3 ou 4 et au moins quatre cycles étant aromatiques lorsque n est 5, au moins l'un des cycles aromatiques contenant un hétéroatome; facultativement substitué par un ou deux substituants; ces substituants ne contenant pas plus de quatre atomes de carbone au total, lorsqu'ils sont pris ensemble, et étant identiques ou différents et étant choisis parmi halogène; cyano; C₁₋₄alcoyle et C₁₋₄alcoxy, chacun facultativement substitué par hydroxyle ou C₁₋₂alcoxy; C₁₋₂alcoyle ou C₁₋₂alcoxy halogéno-substitué; un radical S(O)_{n'}R² où n' est un entier, 0, 1 ou 2 et R² est C₁₋₂alcoyle facultativement substitué par hydroxyle ou C₁₋₂alcoxy; ou R est facultativement substitué par un radical NR³R⁴ ne contenant pas plus de 5 atomes de carbone, où R³ et R⁴ sont identiques ou différents et sont chacun un radical C₁₋₃alcoyle ou NR³R⁴ forme un cycle hétérocyclique de cinq ou six chaînons contenant facultativement un ou deux hétéroatomes supplémentaires;
où R¹ est 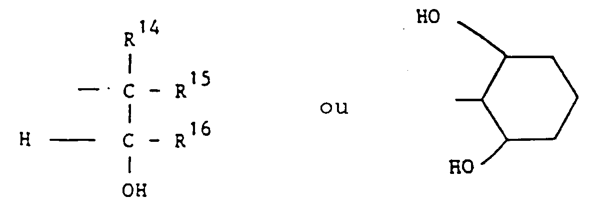 où
R¹⁴ est CH₂OH, CH(CH₃)OH ou CH₂CH₂OH,
R¹⁵ est hydrogène, C₁₋₃alcoyle ou CH₂OH,
R¹⁶ est hydrogène ou méthyle;
de préférence, R¹⁴ est CH₂OH ou CH(CH₃)OH; de préférence R¹⁵ est hydrogène, méthyle, éthyle ou CH₂OH, sauf que R n'est pas un système cyclique tétracyclique contenant 15 à 17 atomes de carbone, trois cycles carbocycliques de six chaînons et un cycle de cinq chaînons dans lequel il y a un hétéroatome,
lequel procédé comprend :
(a) la réduction d'un composé R-CH=N-R¹ ou d'un dérivé convenablement protégé de celui-ci, suivie de la déprotection lorsqu'il y a lieu;
(b) la réduction d'un composé R.CO.NHR¹ dont les radicaux hydroxyle sont facultativement protégés, suivie de la déprotection lorsqu'il y a lieu; et
(c) la réaction d'un composé RCH₂L avec un composé NH₂R¹, où R et R¹ sont tels que définis ci-dessus et L est un radical partant.

2. Procédé suivant la revendication 1 de préparation d'un composé de formule (I), dans lequel un seul ou deux des cycles aromatiques contiennent un hétéroatome.

3. Procédé suivant la revendication 1 ou 2 de préparation d'un composé de formule (I), dans lequel le ou les hétéroatomes sont choisis entre l'azote, le phosphore, l'oxygène, le soufre et le sélénium.

4. Procédé suivant la revendication 3 de préparation d'un composé de formule (I) dans lequel le ou les hétéroatomes sont choisis entre l'oxygène, l'azote et le soufre.

5. Procédé suivant la revendication 1 de préparation d'un composé dans lequel le système cyclique condensé est de formule :
